# EUROPÄISCHE PATENTSCHRIFT

(11) **EP 2 688 465 B1**
(45) Veröffentlichungstag und Bekanntmachung des Hinweises auf die Patenterteilung: **13.05.2015**
(21) Anmeldenummer: 12719241.7
(22) Anmeldetag: 20.03.2012
(51) Int. Cl.: A61B 5/00

(54) **EINRICHTUNG UND VERFAHREN ZUR ERMITTLUNG EINES HAUTENTZÜNDUNGSWERTES**
APPARATUS AND METHOD FOR DETERMINING A SKIN INFLAMMATION VALUE
SYSTÈME ET PROCÉDÉ PERMETTANT DE DÉTERMINER UNE VALEUR D'INFLAMMATION DE LA PEAU

(30) Priorität: 24.03.2011 AT 4202011
(43) Veröffentlichungstag der Anmeldung: 29.01.2014
(73) Patentinhaber: Red.Soft IT-Service GmbH, 7331 Weppersdorf (AT)
(72) Erfinder: SOLDATITSCH, Markus, 7331 Weppersdorf (AT); STROHAL, Robert, 6800 Feldkirch (AT)
(74) Vertreter: Miksovsky, Alexander
(86) Internationale Anmeldenummer: PCT/AT2012/000069
(87) Internationale Veröffentlichungsnummer: WO 2012/126027

(56) Entgegenhaltungen:
- R. V. DOS SANTOS ET AL: "Beyond flat weals: validation of a three-dimensional imaging technology that will improve skin allergy research", CLINICAL AND EXPERIMENTAL DERMATOLOGY, Bd. 33, Nr. 6, 1. November 2008 (2008-11-01), Seiten 772-775, XP55030617, ISSN: 0307-6938, DOI: 10.1111/j.1365-2230.2008.02897.x in der Anmeldung erwähnt
- KIM MIN-GI ET AL: "Objective interpretation of severity of SLS induced edema by stereoimaging.", JOURNAL OF DERMATOLOGICAL SCIENCE AUG 2004 LNKD- PUBMED:15265524, Bd. 35, Nr. 2, August 2004 (2004-08), Seiten 125-131, XP002678367, ISSN: 0923-1811
- PICHE E ET AL: "[FOITS (fast optical in vivo topometry of human skin): new approaches to 3-D surface structures of human skin].", BIOMEDIZINISCHE TECHNIK. BIOMEDICAL ENGINEERING NOV 2000 LNKD- PUBMED:11155533, Bd. 45, Nr. 11, November 2000 (2000-11), Seiten 317-322, XP002678368, ISSN: 0013-5585
- WESTHAUSER M ET AL: "Optimizing color reproduction of a topometric measurement system for medical applications", MEDICAL ENGINEERING & PHYSICS, BUTTERWORTH-HEINEMANN, GB, Bd. 30, Nr. 8, 1. Oktober 2008 (2008-10-01), Seiten 1065-1070, XP025479936, ISSN: 1350-4533, DOI: 10.1016/J.MEDENGPHY.2007.12.014 [gefunden am 2008-02-21]

## Beschreibung

Die Erfindung betrifft eine Einrichtung zur Ermittlung eines Hautentzündungswertes.

Im medizinischen Bereich gibt es bereits die verschiedensten Einrichtungen zur Diagnoseunterstützung für einen Arzt. Dazu sind beispielsweise schon seit langem Röntgenapparate, Computertomographen, diverse 3D-Scanner und vieles mehr im Einsatz.

Im Bereich der Diagnoseunterstützung für Entzündungen auf der Hautoberfläche werden aber zurzeit erst die ersten Schritte gemacht. Dazu geht beispielsweise aus dem Artikel "Beyond flat weals: validation of a three-dimensional imaging technology that will improve skin allergy research" aus dem Fachmagazin "Clinical and Experimental Dermatology, Bd. 33, Nr. 6, 1. November 2008 (2008-11-01), Seiten 772-775, XP55030617, eine Beschreibung einer Methode hervor, wie man mit Hilfe eines 3D-Scanners die Topographie der Hautoberfläche im Bereich von Entzündungen bzw. Hautschwielen messen kann. Durch das dabei entstehende, hoch auflösende dreidimensionale topographische Abbild der Schwiele erhält der diagnostizierende Arzt zusätzliche wichtige Anhaltspunkte. Nachteilig bei diesem System bzw. bei diesem Verfahren ist allerdings, dass lediglich die Höheninformationen und Volumeninformationen in die Diagnose einfließen können. Bei der gemäß diesem Artikel durchgeführten Hautuntersuchung in Form eines sogenannten Pricktests reicht dieser Wert als Diagnoseunterstützung meist auch aus.

Wenn allerdings andere Arten von Hautuntersuchungen durchgeführt werden (z.B. ein sogenannter Epicutantest) reicht der alleinige Höhen- bzw. Volumenwert nicht aus, um eine ausreichende Diagnoseunterstützung für einen diagnostizierenden Arzt zu geben.

Aus Kim Min-Gi et al: "Objective interpretation of severity of SLS induced edema by stereoimaging.", Journal of Dermatological Science Aug 2004 LNKD-PUBMED:15265524, Bd. 35, Nr. 2, August 2004 (2004-08), Seiten 125-131, ist ein abgewandeltes Verfahren zur Evaluierung von Hautentzündungen bekannt geworden.

Piche E et al: "[FOITS (fast optical in vivo topometry of human skin): new approaches to 3-D surface structures of human skin].", Biomedizinische Technik. Biomedical Engineering Nov 2000 LNKD-PUBMED:11155533, Bd. 45, Nr. 11, November 2000 (2000-11), Seiten 317-322, bezieht sich auf eine Analyse von Oberflächenstrukturen menschlicher Haut.

Gemäß Westhauser M et al: "Optimizing color reproduction of a topometric measurement system for medical applications", Medical Engineering & Physics, Butterworth-Heinemann, GB, Bd. 30, Nr. 8, 1. Oktober 2008 (2008-10-01), Seiten 1065-1070, wird auf -eine Optimierung von farblichen Darstellungen für medizinische Anwendungen abgezielt.

Aus der US 2004/0136579 A1 ist ein Verfahren zum Überwachen bzw. zur Quantifizierung des Ausmaßes einer Rötung einer Wunde eines Patienten bekannt geworden, wobei eine Auswertung auf Basis einer Feststellung der Helligkeit und/oder einer Farbkomponente im RGB-Raum zur Feststellung der Abgrenzung einer Wunde vorgenommen wird.

Die Aufgabe der Erfindung besteht daher darin, eine gegenüber dem Stand der Technik verbesserte Diagnoseunterstützung bei Entzündungen der Haut zu schaffen. Insbesondere sollen neben den räumlichen Werten auch aussagekräftige weitere Werte über den gemessenen Entzündungsbereich in die Diagnoseunterstützung einfließen.

Dies wird durch eine Einrichtung zur Ermittlung eines Hautentzündungswertes gemäß Anspruch 1 gelöst.

Gemäß diese Vorrichtung fließen nicht nur die räumlichen Werte in den zu ermittelnden Hautentzündungswert ein, sondern es werden auch zusätzlich die flächenbezogenen und farblichen Werte des gescannten Entzündungsbereiches berücksichtigt. Mit anderen Worten kann durch die vorliegende Erfindung ein viel aussagekräftigerer Wert ermittelt werden, der näher an der tatsächlichen Schwere der Entzündung liegt. Es wird somit die Diagnose wesentlich unterstützt und verbessert und der Arzt muss nicht mehr allein aufgrund seiner subjektiven Einschätzung der Rauheit, der Größe und der Rötung eine Diagnose stellen, sondern kann - basierend auf gespeicherten Erfahrungswerten von vorherigen Messungen und auf den nun tatsächlich gemessenen und mit den Erfahrungswerten vergleichbaren Werten - eine objektivere Diagnose stellen.

Grundsätzlich ist es möglich, den gesamten gescannten Bereich als einen einheitlich zu bewertenden Entzündungsbereich anzusehen. Erfindungsgemäß ist jedoch vorgesehen, dass die Recheneinheit durch Abgrenzung der farblichen Werte der einzelnen Bildpunkte oder durch Abgrenzung der räumlichen Werte der einzelnen Bildpunkte, die flächenbezogenen Werte des aufgenommenen dreidimensionalen Bildes in einen Entzündungsherd und einen an den Entzündungsherd angrenzenden und diesen umgebenden Herdumgebungsbereich unterscheidet. Für die Abgrenzung zwischen Entzündungsherd und Herdumgebungsbereich kann natürlich auch eine Kombination der farblichen Werte, räumlichen Werte und/oder flächenbezogenen Werte herangezogen werden. Das CMYK-Farbmodell ist ein sogenanntes subtraktives Farbmodell, wobei CMYK für Cyan, Magenta, Yellow und Key steht.

Gemäß einem bevorzugten Ausführungsbeispiel der Erfindung kann vorgesehen sein, dass das aufgenommene dreidimensionale Bild des Entzündungsbereiches aus einer Vielzahl von rasterförmig in einem dreidimensionalen Koordinatensystem angeordneten Bildpunkten besteht, wobei jeder flächenbezogene Wert einem einzigen, im Koordinatensystem eindeutigen Bildpunkt entspricht. Die im Koordinatensystem entstehenden Bildpunkte ergeben somit ein virtuelles Abbild der echten Oberfläche der Haut. Bevorzugt kann vorgesehen sein, dass jeder räumliche Wert einem Höhenwert des jeweiligen Bildpunkts im dreidimensionalen Koordinatensystem entspricht.

Um nun ein möglichst aussagekräftiges Ergebnis zu erhalten, ist bevorzugt vorgesehen, dass jedem flächenbezogenen Wert eines durch das opto-elektronische Messgerät aufgenommenen dreidimensionalen Bildes sowohl ein, vorzugsweise einziger, farblicher Wert als auch ein, vorzugsweise einziger, räumlicher Wert zuweisbar ist. Ein einzelner Bildpunkt bzw. ein Pixel kann vorzugsweise die Größe zwischen 1 µm und 10 µm aufweisen. Besonders bevorzugt liegt die Pixelgröße exakt bei 3,05597 µm.

Der HSV-Farbraum ist der Farbräum einiger Farbmodelle, bei dem man den Farbton, die Farbsättigung und den Hellwert bzw. die Dunkelstufe heranzieht.

Die vorliegende Erfindung dient vor allem der Diagnoseunterstützung bei Dermatitis, also einer entzündlichen Reaktion der Haut, vornehmlich der Lederhaut (Dermis). Als Synonym zu Dermatitis kann auch der Begriff Ekzem verwendet werden. Bei den zu untersuchenden Hautentzündungen kann es sich dabei sowohl um natürlich entstandene Entzündungen als auch um absichtlich, durch einen Allergietest hervorgerufene Entzündungen (beispielsweise Epicutantest oder Pricktest) handeln. Es können aber auch Muttermale oder Wunden beurteilt werden, wobei allerdings das Klassifizierungsverfahren entsprechend zu adaptieren ist.

Um nun für die abgegrenzten Bereiche aussagekräftige Detailwerte zu erhalten, die über den gesamten abgegrenzten Bereich gelten, kann bevorzugt vorgesehen sein, dass durch Vergleich der gemittelten farblichen Werte im Entzündungsherd und der gemittelten farblichen Werte im Herdumgebungsbereich ein relativer Gesamt-Farbwert des gesamten Entzündungsherdes ermittelbar ist. Weitere Möglichkeiten bestehen darin, dass aus den räumlichen Werten im Entzündungsherd ein absoluter Gesamt-Volumenwert des gesamten Entzündungsherds ermittelbar ist, und darin, dass durch Vergleich von gemittelten räumlichen Werten im Entzündungherd zu gemittelten räumlichen Werten im Herdumgebungsbereich ein relativer Gesamt-Volumenwert des Entzündungsherdes ermittelbar ist.

Besonders für den letztgenannten relativen Gesamt-Volumenwert kann bevorzugt vorgesehen sein, dass der relative Gesamt-Volumenwert ein Vergleichswert der Oberflächenrauheit im Entzündungsherd zur Oberflächenrauheit im Herdumgebungsbereich ist. Dabei kann sich dass Berechnungsverfahren der Oberflächenrauheit an der Berechnung der Linienrauheit gemäß der deutschen Industrienorm EN ISO 4288 orientieren.

Weitere zusätzliche oder alternative Detailwerte, die für die Berechnung des gesamten Hautentzündungswertes herangezogen werden können, sind im Folgenden genannt. Beispielsweise kann vorgesehen sein, dass ein flächenbezogener Wert einem den Umfang des Entzündungsherdes entsprechenden Umfangswert entspricht und/oder ein flächenbezogener Wert einem die Fläche des Entzündungsherdes repräsentierenden Flächenwert entspricht. Weiters kann vorgesehen sein, dass ein flächenbezogener Wert in Abhängigkeit vom Flächenwert und vom Umfangswert gebildet ist und einem das Verhältnis von Umfangswert zu Flächenwert repräsentierenden Kompaktheitswert entspricht oder dass ein Gesamt-Volumenwert einem die durchschnittliche Höhe aller Erhebungen im Entzündungsherd repräsentierenden durchschnittlichen Höhenwert und/oder einem die Fläche der höchsten Erhebungen repräsentierenden Maximalhöhen-Flächenwert entspricht, wobei die höchsten Erhebungen jene Erhebungen sind, deren Höhe zumindest 70 %, vorzugsweise zumindest 85 %, der Höhe der allerhöchsten Erhebung beträgt.

Weiters kann ein Verfahren zur Ermittlung eines Hautentzündungswertes vorgesehen sein, welches insbesondere mit einer oben genannten Einrichtung durchführbar ist, mit einem opto-elektronischen Messgerät, vorzugsweise einem 3D-Scanner, einer Recheneinheit und einer Anzeigeeinheit, welches folgende Schritte aufweist: Aufnehmen eines dreidimensionalen Bildes eines Entzündungsbereichs auf menschlicher oder tierischer Haut mit dem opto-elektronischen Messgerät, Ermitteln flächenbezogener, farblicher und räumlicher Werte des dreidimensionalen Bildes, Berechnen des Hautentzündungswertes aus den berechneten flächenbezogenen, farblichen und räumlichen Werten, Einteilen des Entzündungsbereichs in einen Entzündungsherd und einen Herdumgebungsbereich, wobei die Fläche des Entzündungsherds von der Fläche des Herdumgebungsbereichs durch die den einzelnen flächenbezogenen Werten zugeordneten farblichen und/oder räumlichen Werten abgegrenzt wird, und Anzeigen des berechneten Hautentzündungswerts auf der Anzeigeeinheit. Dieses Verfahren ist nicht als ein Diagnostizierverfahren anzusehen, sondern als ein Verfahren zur Datenermittlung (farbliche, räumliche und flächenbezogene Werte) oder -verarbeitung, das in einem ärztlich durchgeführten Diagnostizierverfahren verwendbar.

Weitere Verfahrensschritte können Schritte eines Ermittelns des dreidimensionalen Bildes und eines Berechnen des Hautentzündungswertes näher beschreiben bzw. definieren.

Weitere Einzelheiten und Vorteile der vorliegenden Erfindung werden anhand der Figurenbeschreibung unter Bezugnahme auf die in den Zeichnungen dargestellten Ausführungsbeispiele im Folgenden näher erläutert. Darin zeigen:
- Fig. 1: eine schematische Darstellung einer Einrichtung zur Ermittlung eines Hautentzündungswertes,
- Fig. 2 bis 5: Bilder von Hautentzündungsbereichen mit den vier unterschiedlichen Klassen von Hautentzündungswerten,
- Fig. 6 bis 8: den Ablauf des Glättens eines Höhenabbilds, dargestellt im dreidimensionalen Koordinatensystem,
- Fig. 9 und 10: die Darstellung der Höhen in einem Grauwertbild,
- Fig. 11: ein Binärbild der durchschnittlichen Höhen,
- Fig. 12: ein Bild mit einer Höhenbegrenzungskontur,
- Fig. 13: ein Bild mit Prüfung des Schwerpunkts der Höhenbegrenzungskontur,
- Fig. 14: ein Flussdiagramm eines ersten Verfahrens zur Ermittlung eines Hautentzündungswerts,
- Fig. 15 bis 18: die Durchführung einer Konturberechnung auf Basis farblicher und flächenbezogener Werte,
- Fig. 19 bis 22: Schritte zur Werteberechnung bei einem zweiten Verfahren zur Ermittlung eines Hautentzündungswerts und
- Fig. 23: ein Flussdiagramm der wichtigsten Schritte des zweiten beispielhaften Verfahrens zur Ermittlung eines Hautentzündungswertes.

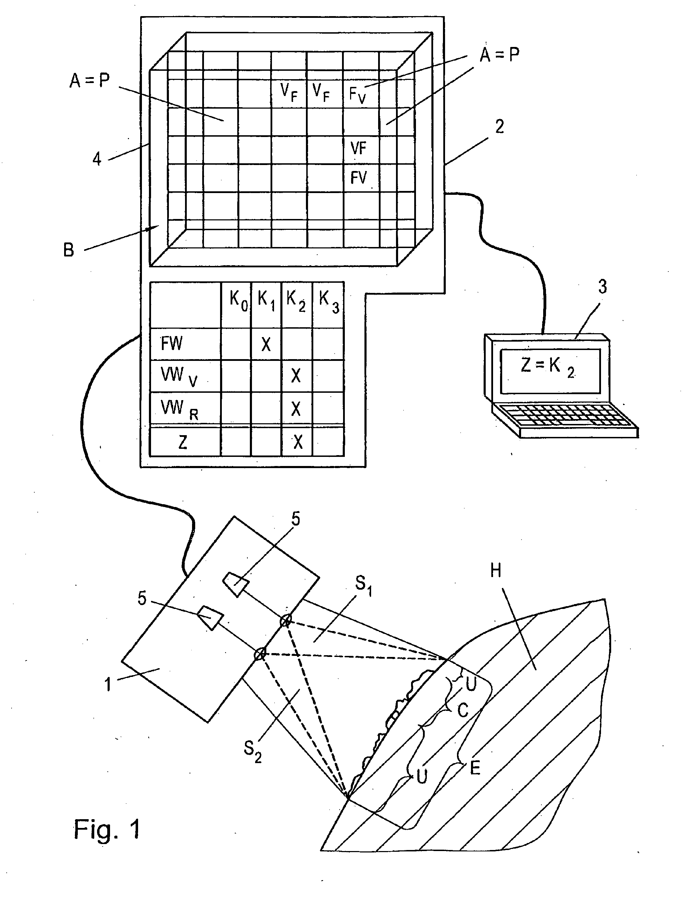

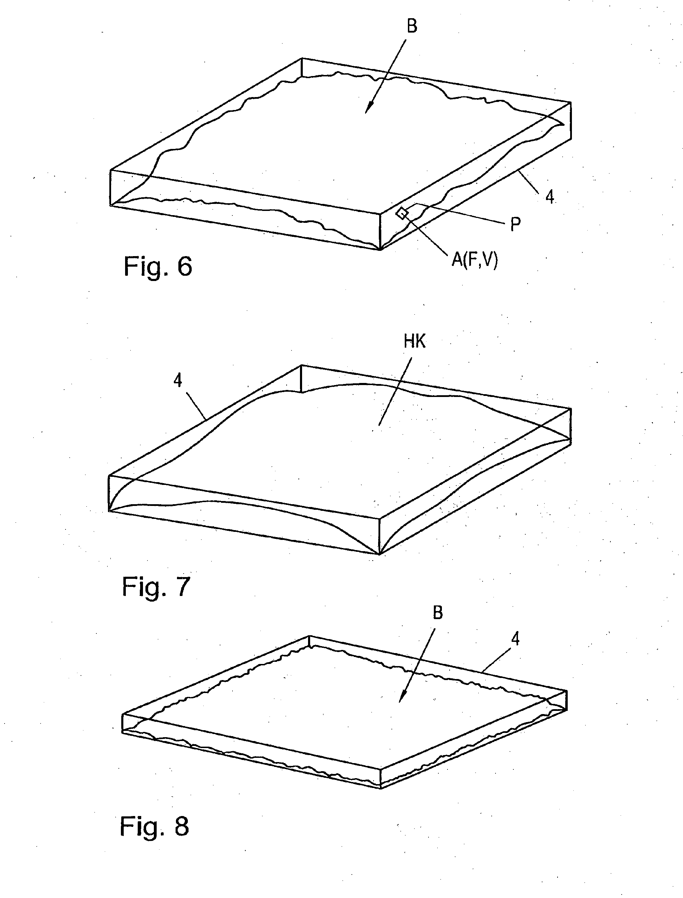

Fig. 1 zeigt die wesentlichen Bestandteile einer Einrichtung zur Ermittlung eines Hautentzündungswertes Z. Dabei wird ein opto-elektronisches Messgerät 1 (3D-Scanner - beispielsweise die PRIMOS pico von GFM) über der Haut H eines Menschen oder eines Tieres gehalten oder bevorzugt direkt auf die Haut H aufgesetzt. Natürlich sollte dabei das Messgerät 1 über einem (vermuteten) Entzündungsbereich E verwendet werden. Durch die einzelnen Scanelemente 5 wird über zwei Scanbereiche S₁ und S₂ der gesamte Entzündungsbereich E aufgenommen und ein entsprechendes dreidimensionales Abbild B an die Recheneinheit 2 übermittelt. Dieses Bild B besteht aus einer Vielzahl von Bildpunkten P, die jeweils einem flächenbezogenen Wert A entsprechen. Jeder einzelne flächenbezogene Wert A ist dabei sozusagen mit einem farblichen Wert F und einem räumlichen Wert V gefüllt. Das gesamte Bild B ist in einem dreidimensionalen Koordinatensystem 4 abgebildet (siehe auch Fig. 6). Die Recheneinheit 2 kann in Form eines Computers ausgebildet sein, der mit dem Messgerät 1 verbunden ist. Die Recheneinheit 2 kann aber auch direkt in das Messgerät 1 integriert sein.

Anhand der gesammelten Werte A, V und F wird dann in einem ersten wichtigen Berechnungsschritt der Entzündungsbereich E in einen Entzündungsherd C und einen Herdumgebungsbereich U unterteilt. Anschließend werden für den Entzündungsherd C absolute Farbwerte FW und/oder absolute Volumenwerte VW und/oder über den gesamten Entzündungsbereich E relative Farbwerte FW und/oder relative Volumenwerte VW_{R} ermittelt. Der relative Farbwert FW kann dabei beispielsweise durch Subtraktion oder Division des gemittelten Magenta-Wertes des Herdumgebungsbereiches U vom gemittelten Magenta-Wert des Entzündungsbereiches C errechnet werden. Der Gesamt-Volumenwert VW kann beispielsweise als absoluter Volumenwert VW_{V} das konkrete Gesamtvolumen der gesamten Schwiele bzw. Entzündung darstellen. Das Bezugszeichen VW_{R} kann für einen relativen Gesamt-Volumenwert stehen, bei dem die Rauheiten des Entzündungsherdes C und des Herdumgebungsbereiches U verglichen werden.

Anschließend kann jeder dieser ermittelten Werte FW, VW_{V} und VW_{R} in eine der Entzündungsklassen K₀, K₁, K₂ oder K₃ eingeteilt werden. Die Grenzen dieser Einteilungsklassen sind dabei vorgegeben und basieren auf in der Recheneinheit 2 gespeicherten, gesammelten und vorher kategorisierten Erfahrungswerten. Aus der Zuteilung zu den einzelnen Klassen K₀, K₁, K₂ und K₃ ergibt sich dann ein gemittelter, vorzugsweise gerundeter, Hautentzündungswert Z, der dann entsprechend auf der Anzeigeeinheit 3 ausgegeben wird. Äquivalent zur optischen Anzeige kann auch eine reine akustische Ausgabe über einen Lautsprecher erfolgen. Als Anzeigeeinheit 3 können auch einzelne Leuchtdioden dienen: Beispielsweise kann der Hautentzündungswert anhand der Farbe einer Diode erkannt werden. Es kann aber auch die Anzahl der leuchtenden Dioden den Hautentzündungswert widerspiegeln.

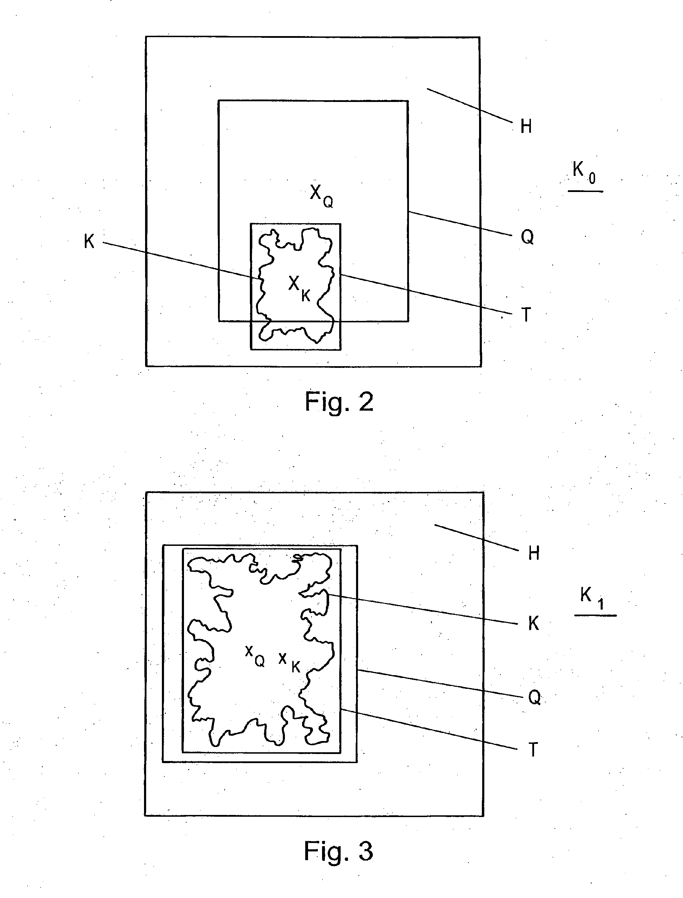

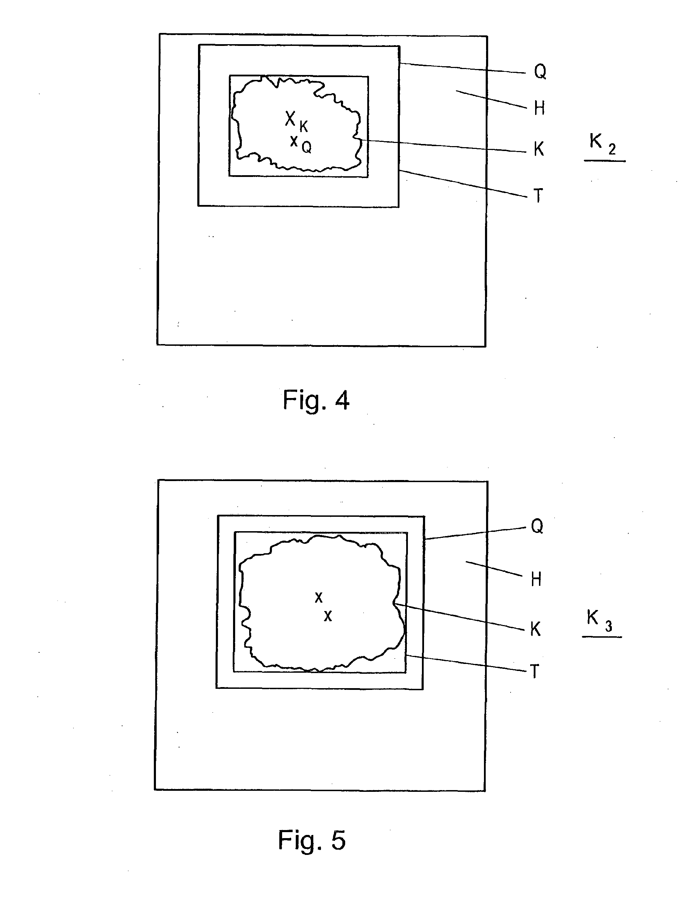

In den Fig. 2 bis 5 sind beispielhaft Bilder von unterschiedlichen Entzündungsbereichen E gezeigt, wobei jeweils ein Segmentierungsrechteck Q und ein Konturumgebungsrechteck T eingezeichnet ist. Die Kontur K bildet die Grenze zwischen Entzündungsherd C und Herdumgebungsbereich U. Zudem ist auf jedem dieser Fig. 2 bis 5 das Zentrum X_{Q} des Segmentierungsrechtecks Q und der Schwerpunkt X_{K} der Kontur K dargestellt. Als jeweiliger exakter Punkt ist der Kreuzungspunkt des Buchstabens X anzusehen. In Fig. 5 ist eine intensive Rötung und Schwellung mit großen Blasen dargestellt (Entzündungsklasse K₃), bei dem das linke obere X dem Zentrum des Segmentierungsrechtecks Q und das rechte untere X dem Schwerpunkt der Kontur K entspricht.

Im Folgenden sind zwei Verfahren zur Ermittlung eines Hautentzündurigswertes detailliert dargelegt, dabei soll es allerdings nicht ausgeschlossen sein, dass einzelne oder mehrere Berechnungsschritte der beiden Verfahren auch in einem eigenen Verfahren mit beliebig "gemischten" Berechnungsschritten durchgeführt werden. Natürlich können bei jedem einzelnen Verfahren auch teilweise Verfahrensschritte weggelassen werden. Wesentlich ist, dass für die Berechnung des Hautentzündungswertes jeweils flächenbezogene, räumliche und farbliche Werte A, V und F des mit dem opto-elektronischen Messgerät aufgenommenen dreidimensionalen Bildes B berücksichtigt werden. Natürlich soll auch nicht ausgeschlossen sein, dass noch andere nicht genannte alternative Berechnungsvarianten für die Ermittlung eines Hautentzündungswertes Z herangezogen werden können.

Dementsprechend wird im Folgenden ein erstes Verfahren mit beispielhaften Algorithmen für einen Epicutantest beschrieben. Die Analyse der Epicutantests gliedert sich dabei in drei Schritte:
i) Erkennung der Quaddel (Schwiele) mittels eines Höhen Segmentierungsverfahrens
ii) Vermessung der Quaddel (Höhen- und Farbwerte)
iii) Bewertung der Messergebnisse

Diese drei Schritte werden im nachfolgenden Text beschrieben, wobei die Probleme der bisher eingesetzten Lösung dargelegt und Möglichkeiten aufgezeigt werden, wie eine für die Erfindung geeignete, neue Softwarelösung den Ordinationsablauf noch besser und effizienter unterstützen kann.

Durch eine Neu-Implementierung der Softwarelösung können bestehende Probleme bereits vorab verhindert werden und die Struktur der Applikation an die derzeitigen Erfordernisse optimal angepasst werden. Weiters besteht die Möglichkeit Optimierungen in den einzelnen Bereichen durchzuführen und dadurch den gesamten Unterstützungsprozess effizienter zu gestalten und den zeitlichen Aufwand für die Benutzer des Systems zu reduzieren.

### i) Höhen-Segmentierung:

Die Segmentierung gliedert sich grob in 7 Schritte
1. Glätten des Höhenabbildes
2. Filterung des Höhenabbildes
3. Darstellung der Höhen in einem Grauwert-Bild (Maximalhöhe ist weiß, Minimalhöhe ist schwarz)
4. Ermittlung der überdurchschnittlichen Magenta-Werte im CMYK-Bild und Erhöhung der Werte im Höhen-Grauwertbild an jenen Stellen, die einen überdurchschnittlichen Magenta-Wert haben.
5. Berechnung der durchschnittlichen Höhe und Erstellung eines Binärbildes.
6. Finden der Begrenzungskontur der größten zusammenhängenden Erhebung.
7. Prüfung, ob das center of gravity (Schwerpunkt) des Höhen-Grauwertbildes innerhalb des Rechtecks liegt, welches die gefundene Begrenzungskontur der größten zusammenhängenden Erhebung umschließt.

### 1. Glätten des Höhenabbildes:

Da jene Hautstelle, die erfasst wurde (siehe Originalhöhenbild gemäß Fig. 6), in den meisten Fällen eine Krümmung aufweist, wird das Höhenabbild weitestgehend begradigt, sodass mit einer idealisierten ebenen Haut weiter gearbeitet werden kann.

Dazu werden jeweils an den Rändern die 25 äußersten Höhenwerte herangezogen und mit ihrer Hilfe eine gekrümmte Ebene berechnet, die der Hautkrümmung HK entspricht (siehe Fig. 7).

Das neue Höhenabbild wird nun wie folgt gebildet: Jene Werte, die im originalen Höhenabbild kleiner sind als der korrespondierende Wert der berechneten Ebene, werden auf den Wert der berechneten Ebene gesetzt. Alle anderen Werte behalten die Originalwerte bei. Im Anschluss wird von jedem Höhenwert der korrespondierende Wert der berechneten Ebene abgezogen. Auf diese Weise werden die Wölbung der Haut, sowie eventuelle Hautporen, die tiefere Täler im Höhenabbild erzeugen, eliminiert. Die Höhe 0 kann nun als Basishöhe der Haut betrachtet werden. Ein derart geglättetes Höhenabbild (Originalhöhenbild abzüglich der berechneten Hautkrümmung) ist in Fig. 8 dargestellt.

### 2. Filterung des Höhenabbildes:

Um kleinere Ausreißer im Höhenabbild zu eliminieren wird dieses mit einem Medianfilter geglättet (momentan wird mit der Nachbarschaftsgröße 3 gearbeitet).

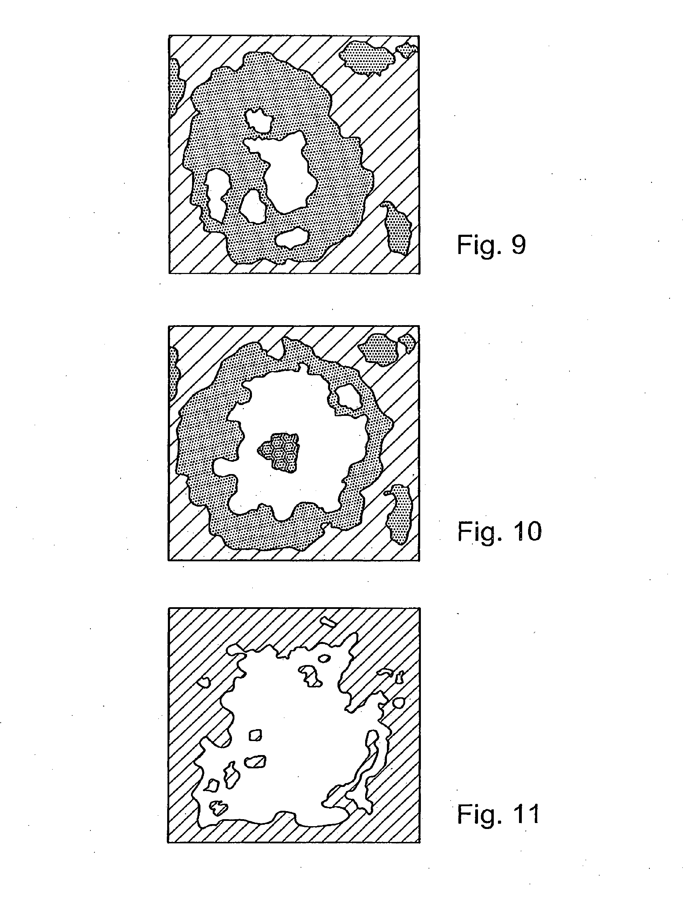

### 3. Darstellung der Höhen in einem Grauwert-Bild (Fig. 9):

Für die weitere Verarbeitung des Höhenabbildes mit Hilfe von Bildverarbeitungsalgorithmen wird aus dem Höhenabbild ein Grauwert-Bild mit 256 Graustufen berechnet. Die höchste Höhe wird für den Wert 255 (weiß), die niedrigste Höhe wird für den Wert 0 (schwarz) herangezogen. Die Höhenwerte dazwischen werden proportional dazu in verschiedene Graustufen umgerechnet.

### 4. Erhöhung der Werte im Höhen-Grauwertbild an jenen Stellen, die einen überdurchschnittlichen Magenta-Wert haben (Fig. 10):

Um jene Stelle besser eingrenzen zu können die eine Entzündung abbilden, werden diese im Höhenabbild um den Grad der überdurchschnittlichen Rötung erhöht. Dazu wird das Originalbild in ein CMYK-Bild umgerechnet und der Magenta-Kanal betrachtet. Es wird ein Grauwert-Bild erstellt, das dem Magenta-Kanal entspricht, jedoch werden alle Magenta-Werte, die nicht einen bestimmten Prozentsatz (beispielsweise 120%) des durchschnittlichen Magenta-Wertes erreichen, auf 0 gesetzt.

Danach werden die einzelnen Punkte des Höhenabbildes betrachtet und mit dem entsprechenden Pixel im Magenta-Bild verglichen. Ist der Wert im Magenta-Bild höher als jener im Grauwert-Bild des Höhenabbildes, so wird der Pixel im Grauwert-Bild des Höhenabbildes aus einem Anteil des aktuellen Wertes und einem Anteil des Wertes des Magenta-Bildes neu berechnet (beispielsweise trägt der Wert des Magenta-Bildes 60% und der Wert des Höhen-Grauwert-Bildes 40% zum neuen Wert bei).

### 5. Berechnung der durchschnittlichen Höhe und Erstellung eines Binärbildes (Fig. 11):

Aus dem mit Hilfe des Magenta-Kanales des CMYK-Bildes verstärkten Grauwert-Bild des Höhenabbildes wird nun ein Binärbild berechnet, welches zur Suche von Konturen benötigt wird. Als Threshöld wird dabei der durchschnittliche Grauwert (mal einem Koeffizienten, aktuell 2.0) angenommen.

Bevor das Binärbild erstellt wird, wird das Grauwert-Bild noch mit einem Median-Filter geglättet (Die aktuelle Nachbarschaftsgröße beträgt 9). Das Binärbild wird noch erodiert und geweitet (aktuell werden drei Iterationen des Erodierens und eine des Weitens durchgeführt).

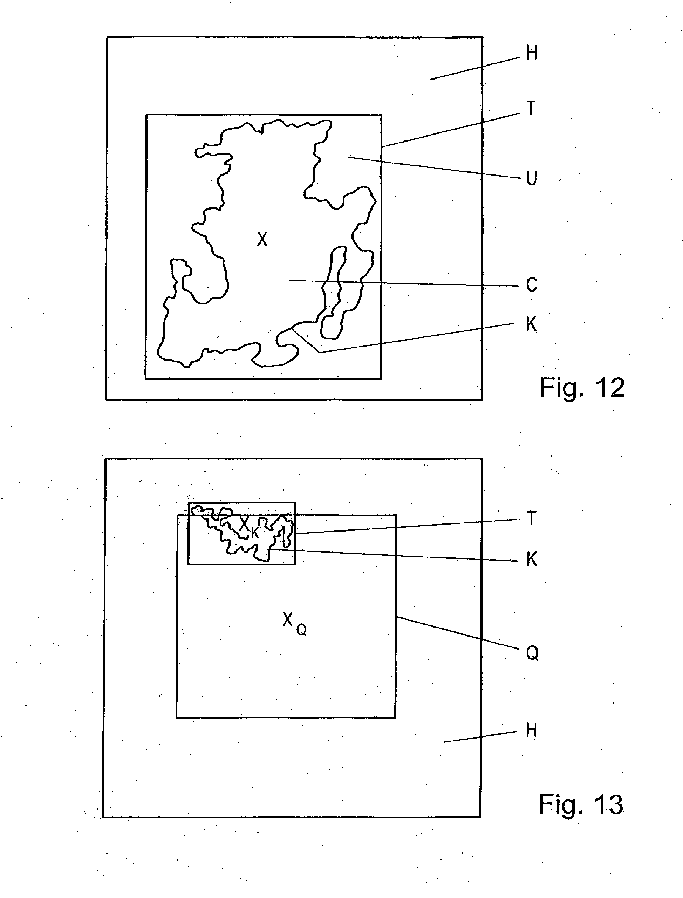

### 6. Finden der Begrenzungskontur der größten zusammenhängenden Erhebung (Fig. 12):

Jene Teile des Höhenabbildes, die über der Durchschnittshöhe (mal einem Koeffizienten) liegen, sind in diesem Binärbild als weiße Flecken abgebildet. Der Algorithmus sucht nun jenen weißen Fleck im Binärbild, der die größte Fläche besitzt und liefert die Begrenzungskontur K des Bereiches, sowie ein Begrenzungsrechteck T, welches die Kontur K umschließt. Der von der Kontur K umschlossene Bereich (Entzündungsherd C) fängt jenen Teil im Höhenabbild ein, der die höchste geschlossene, entzündete Erhebung und somit die gesuchte Hautschwellung darstellt und wird vom Herdumgebungsbereich U umgeben.

### 7 Berechnung und Prüfung des center of gravity (Schwerpunkt) des Höhen-Grauwertbildes (Fig. 13):

Als Kontrollmaßname wird das "center of gravity" des Höhen-Grauwertbildes berechnet (Punkt X_{Q}). Liegt das "center of gravity" innerhalb des Bereich der gefundenen Begrenzungskontur K bzw. dem dieser Kontur K umschließenden Rechteck T, so bestätigt dies die gefundene Kontur K und somit die Lokalisation der vermeintlichen Messfläche.

Liegt das "center of gravity" nicht innerhalb des Rechtecks T wie in Fig. 13, so kann davon ausgegangen werden, dass die gefundene Erhebung im Vergleich zu anderen Erhebungen nicht herausragend ist. Im Regelfall handelt es sich dabei um jene Tests die keine oder eine unterdurchschnittlich ausgeprägte Schwellung aufweisen.

In diesem Fall wird für die weitere Vermessung nicht jener Bereich herangezogen, der die Kontur K umschließt, sondern jener Bereich der die vermeintliche Messfläche bzw. das diesen Bereich umschließende Quadrat Q darstellt. Den Mittelpunkt X_{Q} des Quadrats Q stellt der "center of gravity" des Höhen-Grauwertbildes dar (die Größe des Quadrats entspricht jeweils der realen Messfläche).

### ii) Vermessung:

Nachdem die Erkennung der Hautschwellung abgeschossen wurde, wird diese vermessen. Dabei werden drei Kennwerte ermittelt, welche zur Bewertung herangezogen werden:
1. Das Volumen der Schwellung im Vergleich zur Fläche der Schwellung
2. Die Rauheit der Schwellung im Vergleich zur Rauheit der restlichen Hautfläche
3. Die Rötung der Schwellung im Vergleich zur restlichen Hautfarbe

### 1. Das Volumen der Schwellung im Vergleich zur Fläche der Schwellung:

Die Grundfläche der Schwellung ist jener Bereich, der von der Kontur K umschlossen wird. Es wird nun das Gesamtvolumen der Schwellung berechnet, die sich innerhalb der Kontur K befindet. Hierbei wird nur jener Teil der Höhe gezählt, der über der Durchschnittshöhe der Haut liegt.

Dieses errechnete Gesamtvolumen der Schwellung wird durch die Fläche dividiert. Das Ergebnis ist die Durchschnittshöhe der Schwellung. Diese wird für die Bewertung herangezogen.

### 2. Die Rauheit der Schwellung im Vergleich zur Rauheit der restlichen Hautfläche:

Eine weitere signifikante Kenngröße der Schwellung stellt die Rauheit dar. Damit eine mögliche raue Normalhaut nicht zu sehr die Messergebnisse beeinflusst, wird die Rauheit innerhalb und außerhalb des Rechtecks berechnet, welches die Begrenzungskontur umschließt. Zur Bewertung wird nun die Rauheit der Schwellung (innerhalb des Recktecks) abzüglich der Rauheit der restlichen Haut (außerhalb des Rechtecks) herangezogen.

Das zur Berechnung der Oberflächenrauheit implementierte Verfahren orientiert sich am Verfahren zur Berechnung der Linienrauheit (DIN EN ISO 4288).

Als Begrenzungsparameter werden 10% bzw. 90% herangezogen. Das heißt, dass nicht die Differenz zwischen dem höchsten Punkt (0% Flächenmaterialanteil) und dem niedrigsten Punkt (100% Flächenmaterialanteil) als Rauheitswert herangezogen wird, sondern die Differenz zwischen jenen Schnitthöhen, die einen Flächenmaterialanteil von 10% bzw. 90% ergeben.

### 3. Die Rötung der Schwellung im Vergleich zur restlichen Hautfarbe:

Zusätzlich zu den zwei Messwerten, die aus dem Höhenabbild berechnet werden, wird aus dem Farbbild der Grad der Rötung der Messfläche ermittelt. Dazu wird der Magenta-Kanal der CMYK-Darstellung des Originalfarbbildes der Messstelle herangezogen.

Ähnlich wie bei der Berechnung der Rauheit und des Durchschnittsvolumens wird auch hier jeweils ein Wert innerhalb und ein Wert außerhalb des Bereiches ermittelt, der von der Kontur begrenzt wird. Zur weiteren Bewertung wird der Durchschnittswert innerhalb der Kontur abzüglich des Durchschnittswertes außerhalb der Kontur verwendet.

### iii) Bewertung:

Die Quaddel wird, nachdem sie vermessen wurde, bewertet und in die in der Praxis üblichen vier Klassen eingeteilt. Die nachfolgende Tabelle enthält eine ungefähre, subjektive Beschreibung der Klassen.

| **Klasse** | **Beispiel** | **Beschreibung** |
|---|---|---|
| 0 (K₀) | Fig. 2 | Zweifelhafte Reaktion: Eventuell eine leichte Rötung |
| 1 (K₁) | Fig. 3 | Schwach positive Reaktion: Rote und leicht geschwollene Haut |
| 2 (K₂) | Fig. 4 | Starke positive Reaktion: Rote und geschwollene Haut mit einzelnen Bläschen |
| 3 (K₃) | Fig. 5 | Extrem positive Reaktion: Intensive Rötung und Schwellung mit großen Blasen |

Die Gesamtbewertung der Quaddel setzt sich aus den einzelnen Teilbewertungen der Kennwerte aus der Vermessung zusammen. Im aktuellen Fall werden drei Teilbewertungen in vier Klassen erstellt, deren gerundeter Mittelwert die Klasse der Gesamtbewertung ergibt. Da der Wert der Errötung bei stark geröteter Normalhaut ab einen definierten Grenzwert an Aussagekraft abnimmt, wird im Zuge der Bewertung explizit auf diesen Umstand eingegangen.

Sollte eine Hautrötung der Normalhaut über dem Grenzwert festgestellt werden, so wird die Errötung der Schwellung nicht zur Bewertung herangezogen.

Zum besseren Verständnis dient das nachfolgende beispielhafte Rechenbeispiel:

| | **Beispielklassengrenzen** | **Beispielmesswerte** | **Klassen der Teilbewertung** |
|---|---|---|---|
| Volumen | 0-5,5-11,11-16, 16-unendlich | 6 | 1 |
| Rauheit | 0-3,3-13,13-17,17-unendlich | 14 | 2 |
| Errötung zwischen 0 und 1 | 0-4,4-9,9-11,11-unendlich | 12 | 3 |

Daraus ergibt sich die Gesamtbewertung 2 (Mittelwert von 1+2+3 ist 2).

### Rechenbeispiel bei starker Hautrötung:

| | **Beispielklassengrenzen** | **Beispielmesswerte** | **Klassen der Teilbewertung** |
|---|---|---|---|
| **Volumen** | 0-5,5-11,11-16, 16-unendlich | 19 | 3 |
| **Rauheit** | 0-3,3-13,13-17,17-unendlich | 18 | 3 |
| **Errötung zwischen 0 und 1** | 0-4,4-9,9-11,11-unendlich | 2 | 1 |

Die Klassifizierung der Errötung würde das Gesamtergebnis auf 2 (Gerundeter Mittelwert von 3+3+1 ist 2) senken. Durch die Berücksichtigung der Hautrötung über dem Grenzwert ergibt sich eine Klassifikation von 3 (Mittelwert von 3+3 ist 3).

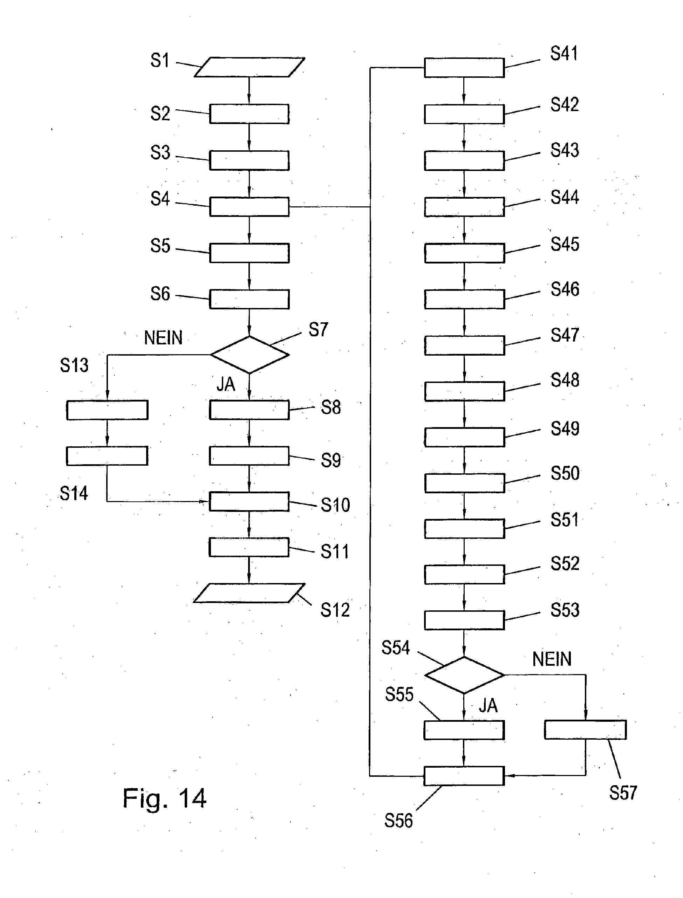

Fig. 14 zeigt ein Flussdiagramm des ersten Verfahrens und stellt die eben erwähnten Verfahrensschritte nochmals in einem logischen Zusammenhang dar.

Um nicht nur allgemeine Bereiche für die Entzündurigsklassen K₀ bis K₃ anzugeben, sind im Folgenden vier, unterschiedlichen Klassen zugehörende, konkrete Beispiele von Messwerten samt Bewertung angegeben. Diese beziehen sich konkret auf die in den Fig. 2 bis 5 dargestellten unterschiedlichen Grade bzw. Klassen von Entzündungen.

### i) Vermessung

Zu diesen Bildern wurden folgende Kennwerte ermittelt:
1. Durchschnittshöhe (Durchschnittsvolumen) der Entzündung
2. Rauheitswert abzüglich der Grundrauheit (relative Rauheit)
3. Rötung im Vergleich zur Farbe der übrigen Haut (relative Rötung)

Sollte das center of gravity X_{Q} des Segmentierungsbildes nicht im Rechteck T liegen, das die Kontur K umschließt, so wird bei der Vermessung anstatt der Bereiche innerhalb bzw. außerhalb der Kontur K und innerhalb bzw. außerhalb des Rechtecks T, das die Kontur K umschließt, jener Bereich verwendet, der innerhalb bzw. außerhalb des Quadrates Q ist, das mit dem center of gravity X_{Q} des Segmentierungsbildes als Mittelpunkt gebildet wird.

### 1. Durchschnittshöhe (Durchschnittsvolumen) der Entzündung:

Die Höhen aller Messpunkte, die sich innerhalb der erkannten, durch die Kontur K begrenzten Quaddel befinden, werden aufsummiert. Hierbei wird nur jener Teil der Höhe gezählt, der über der Durchschnittshöhe der Haut liegt. Dieses Volumen wird durch die Anzahl der Messpunkte dividiert. Das dadurch berechnete Durchschnittsvolumen wird zur Bewertung herangezogen.

Werte der Beispielbilder (Die Grundfläche eines Pixels beträgt 0,00305597 mm²):

| | **Gesamtvolumen der Quaddel** | **Gesamtfläche der Quaddel** | **Durchschnittsvolumen pro Pixel** |
|---|---|---|---|
| **Fig. 2** - **- K₀** | 1,6913977 mm³ | 190,9985264 mm² | 0,0000271 mm³ |
| **Fig. 3** **- K₁** | 7,3151578 mm³ | 61,7780914 mm² | 0,0003619 mm³ |
| **Fig. 4** **- K₂** | 16,6766525 mm³ | 62,867547 mm² | 0,0008106 mm³ |
| **Fig. 5** **- K₃** | 40,2830175 mm³ | 93,6228937 mm² | 0,0013149 mm³ |

### 2. Rauheitswert abzüglich der Grundrauheit (relative Rauheit):

Die Rauheit der Oberfläche wird für die Fläche innerhalb der Kontur K und für die Fläche zwischen der Kontur K und dem Rechteck T berechnet. Die Differenz zwischen den beiden Rauheitswerten bildet eine Bewertungsgrundlage.

### Werte der Beispielbilder:

| | **Rauheit innerhalb des Begrenzungsrechtecks** | **Rauheit außerhalb des Begrenzungsrechtecks** | **Differenz** |
|---|---|---|---|
| **Fig. 2** **- K₀** | 0,0732433 mm | 0,0550084 mm | 0,0182349 mm |
| **Fig. 3** **- K₁** | 0,1658371 mm | 0,0924609 mm | 0,0733762 mm |
| **Fig. 4** **- K₂** | 0,3263570 mm | 0,1401592 mm | 0,1861978 mm |
| **Fig. 5** **- K₃** | 0,4609349 mm | 0,1506546 mm | 0,3102803 mm |

### 3. Rötung im Vergleich zur Farbe der übrigen Haut (relative Rötung):

Aus dem Magenta-Kanal des Farbbildes wird die durchschnittliche Rötung der Bereiche innerhalb und außerhalb der erkannten Quaddel (Kontur K) ermittelt. Die Differenz der beiden Durchschnittswerte fließt in die Bewertung ein.

### Werte der Beispielbilder:

| | **Rötung der Quaddel** | **Rötung der Umgebung** | **Differenz** |
|---|---|---|---|
| **Fig. 2** **- K₀** | 65,104384 | 66,6805231 | -1,5761391 |
| **Fig. 3** **- K₁** | 75,2352490 | 67,1557576 | 8,0794914 |
| **Fig. 4** **- K₂** | 137,7521633 | 107,7737394 | 29,9784239 |
| **Fig. 5** **- K₃** | 104,6286619 | 67,2027139 | 37,425948 |

### 3a. Rötung der Umgebungshaut

Ist der durchschnittliche Wert der Rötung der Haut außerhalb der Quaddel über einem Schwellwert, so wird die relative Rötung nicht zur Bewertung herangezogen.

### Werte der Beispielbilder:

Klasse 0: 66,6805231
Klasse 1: 67,1557576
Klasse 2: 107,7737394
Klasse 3: 67,2027139

### ii) Bewertung:

Die Bewertung erfolgt zunächst für jeden Wert separat. Dazu werden für jeden Messwert Grenzwerte festgelegt. Die derzeit verwendeten (aber individuell in Absprache mit Ärzten festlegbaren und veränderbaren) Grenzwerte für die einzelnen Messwerte sind wie folgt:

| | **Klasse 0** | **Klasse 1** | **Klasse 2** | **Klasse 3** |
|---|---|---|---|---|
| **Durchschnittsvolumen der Entzündung** | <0,000225492 | <0,000646506 | <0,001029246 | >=0,001029246 |
| **Rauheitswert abzüglich der Grundrauheit** | <0,02655275 | <0,1102376 | <0,21623841 | >=0,21623841 |
| **Rötung im Vergleich zur Farbe der übrigen Haut** | <4,68131157 | <12,48887981 | <32,98374644 | >=32,98374644 |

Zusätzlich gibt es noch einen Schwellwert, welcher festlegt, ab welcher Rötung die Umgebungshaut als zu rötlich angesehen wird, und bestimmt, ob die Rötung im Vergleich zur Farbe der übrigen Haut zur Bewertung herangezogen wird. Dieser Schwellwert ist momentan mit 109,98770675 festgelegt.

Die Gesamtklassifizierung ergibt sich aus der (gerundeten) Durchschnittsklassifizierung der Teilbewertungen.

Im Folgenden wird eine zweite Variante zur Ermittlung eines Hautentzündungswerts Z angegeben, die mit der erfindungsgemäßen Einrichtung durchführbar ist.

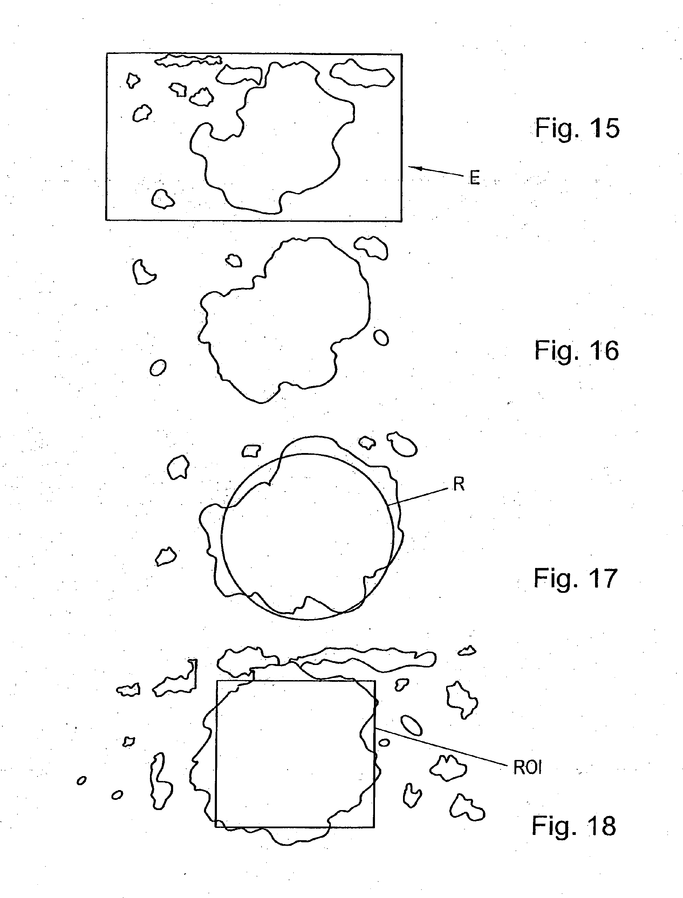

Durch Analyse verschiedenster Farbräume und Darstellungen hat sich herausgestellt, dass sich der Magenta-Farbraum in der CMYK-Falschfarbendarstellung und der Sättigungswert im HSV-Farbraums am Besten für die Filterung und Bestimmung von Entzündungsherden auf der menschlichen Haut eignen. Am Beginn der Bildverarbeitung werden dazu zwei Bilder aus dem Originalbild (Fig. 15) generiert und es erfolgt eine Konvertierung in ein CMYK- und ein HSV-Bild.

Als nächster Schritt kann optional eine Vorfilterung des Bildes, in welcher Pflaster ausgefiltert werden, durchgeführt werden. Dazu wird anhand eines fixen Grenzwertes in der Magenta-Ebene des CMYK-Bildes für jeden Pixel unterschieden, ob der Bildpunkt einem Pflaster oder der Haut entspricht (=sogenannte Threshold-Funktion). Als Startwert wird für den Grenzwert 100 angenommen, beim Durchlaufen jedes Pixels des Bildes wird also überprüft, ob der Magentawert höher ist als 100 oder nicht. Ist das der Fall, wird der Pixelwert aus dem Originalbild übernommen, falls nicht, wird der Farbwert auf 0 (=schwarz) gesetzt. Danach wird mittels einer Bewertungsfunktion überprüft, ob ausreichend Bildpunkte für die weitere Verarbeitung erhalten geblieben sind, oder ob der Threshold zu hoch angesetzt wurde. In diesem Fall erfolgt eine Abstufung des fixen Grenzwerts und die Filterung und Kontrolle wird erneut durchgeführt. Dieser Prozess wird bis zu viermal wiederholt, um zu gewährleisten, dass eine optimal Filterung von Pflastersegmenten im Bild erfolgt, ohne dass dabei zu viel der eigentlichen Information verloren geht.

Nun wird das Bild im Magenta-Farbraum gefiltert. Dazu existieren zwei Varianten, die abhängig von der eingesetzten Kamera verwendet werden. Bei der ersten wird der Magenta-Mittelwert aller erhaltenen Pixel berechnet. Danach wird in einer Schleife erneut mittels einer Threshold-Funktion (mulfaktor) ausgefiltert, ob ein Bildpunkt einer Entzündung oder neutraler Haut zugeordnet werden kann. Dabei wird der Thresholdwert iterativ verringert, d.h. also im ersten Schritt werden alle Pixel, die über einem gewissen Prozentsatz des durchschnittlichen Magentawerts liegen, übernommen. In der zweiten Variante wird nicht der eigentliche Pixelwert mit dem durchschnittlichen Magentawert*mulfaktor verglichen, sondern ein Mittelwert aus der 5x5 Pixel Umgebung des Pixels mit dem Thresholdwert verglichen. Das Ergebnisbild ist in beiden beiden Fällen ein Grauwertbild mit den gefilterten Magentapixeln. Daraufhin folgen einige Bildverarbeitungsschritte um das Filterungsergebnis zu optimieren. Dazu zählt ein Mittelwertfilter (um Pixelrauschen zu eliminieren, dh kleine Pixelgruppen werden ausgefiltert). Erode- und Dilate-Funktionen werden dann noch dazu verwendet, um etwaige Lücken zu schließen. Danach erfolgt eine Konvertierung in ein Binärbild (=Schwarz/Weiß-Bild), in welchem nun ein Konturfindungs-Algorithmus durchgeführt wird. Die gefundenen Konturen werden dann Schritt für Schritt untersucht, um eine sogenannte Region of Interest ROI, als einen potentiellen Segmentierungsbereich, identifizieren zu können (siehe Fig. 16). Dazu wird, falls die Kontur einer Mindestgröße und einer definierten Position im Bild entspricht, zunächst die Kompaktheit der Kontur (=Fläche der Kontur/Umfang der Kontur) berechnet. Je regelmäßiger die Kompaktheit, desto eher kann von einer Entzündung ausgegangen werden. Ist diese größer als die Kompaktheit einer vorangegangenen Kontur, so wird für die aktuelle Kontur der durchschnittliche Radius R (ergibt sich aus der Distanz jeden Bildpunkts der Kontur zum Schwerpunkt X_{K} der Kontur gemittelt über den Umfang der Kontur ermittelt). Dadurch ergibt sich ein Kreis mit Zentrum im Schwerpunkt und einem Radius = gemittelter Radius (siehe Fig. 17), das umgebende Quadrat wird als Region of Interest ROI definiert (siehe Fig. 18).

In diesem Bereich erfolgt nun für die Bewertung der Segmentierung eine Ermittlung des durchschnittlichen Magentawerts sowie des durchschnittlichen Sättigungswerts. Als weiterer Beurteilungskoeffizient wird der Kompaktheitswert durch den mittleren Radius dividiert (da die durchschnittliche Größe des gefilterten Bereichs für die Klassifizierung eine wesentliche Rolle spielt).

Somit entstehen am Ende der Berechnung für einen gewissen Threshold drei Klassifizierungswerte. Danach wird der Threshold(mulfaktor) um 1 % reduziert und die Berechnung erneut durchgeführt. Dies geschieht im ersten Schritt zehn Mal. In den erhaltenen Werten wird dann anhand des Maximalwerts dieser Kalkulationen die optimalste Region für die Weiterverarbeitung in Betracht gezogen. Wird im ersten Schritt kein geeignetes Ergebnis erzielt, erfolgt eine weitere Reduzierung in 10 Schritten des Threshold-Werts. Das Ergebnis der ersten Schritte (vorfilterung, Filterung, Berechnung der Region of Interest - siehe Fig. 18) ist ein Quadrat mit definiertem Anfangspunkt und einer definierten Seitenlänge in Pixel, sowie die Klassifizierungswerte durchschnittlicher Magentawert (1. Klassifizierungswert aus Segmentierung), durchschnittlicher Sättigungswert (2. Klassifizierungswert) sowie die Kompaktheit bezogen auf den Radius (kompaktRadius, 3. Klassifizierungswert).

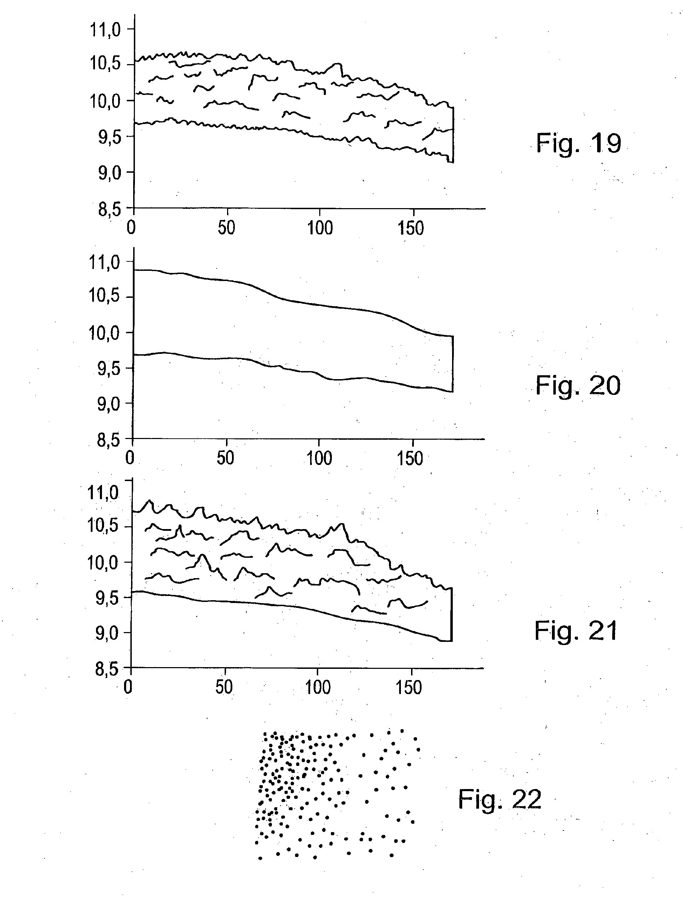

Das segmentierte Quadrat wird dann den Höhenverarbeitungsalgorithmen zur weiteren Verarbeitung und Ermittlung der Kennwerte übergeben. Der Ablauf der Höhenermittlung ist in den Fig. 19 bis 22 dargestellt.

Dabei wird im ersten Schritt für die Region of Interest aus der Original-Höhenmap erstellt, die sich aus der Aufnahme mit der GFM-Kamera ergibt (Für jeden Bildpunkt existiert eine absolute Höheninformation, siehe Fig. 19) gefiltert. Dies geschieht mit Hilfe eines Mittelwertfilters, der solange auf die Höhenmap angewandt wird, bis sich eine homogene Fläche ergibt, welche eine Art Mittelfläche für die gesamte Aufnahme darstellt (siehe Fig. 20). Mit Hilfe dieser Mittelfläche ist es nun möglich, relative Höhen der einzelnen Spitzen im Höhenbild zu bestimmen. Dazu wird zunächst eine Subratktions-Höhenmap erstellt, indem die Mittelfläche von der Originalfläche subtrahiert wird (siehe Fig. 21). Für alle übriggebliebenen Pixel im Bild wird nun die relative Höhe addiert, was ein durchschnittliches Volumen ergibt (= erster Höhen-Klassifzierungswert.AvgVolume).

Im nächsten Schritt werden alle Spitzen (mit dem höchsten Höhenwert GH wird begonnen) gesucht und in einer Liste erfasst. Wird eine neue Spitze als der Maximalwert in der verbliebenen Höhenmap gefunden, wird sowohl in positive als auch in negative x- und y-Richtung solange gesucht, bis die Pixelwerte das erste mal wieder steigen. Es wird also eine Art Gipfelfläche von der Gipfelspitze G aus ermittelt. Die dadurch erhaltene Fläche wird aus der Map gelöscht um die Suche nach dem nächsthöheren Gipfel zu ermöglichen. Dieser Vorgang wird solange wiederholt, bis keine Spitzen mehr gefunden werden, dabei werden für alle gefundenen Spitzenwerte die Höhen gespeichert, am Ende dieses Vorgangs wird dann die Gesamthöhe durch die Anzahl der gefundenen Spitzen dividiert um eine durchschnittliche relative Höhe aller Spitzen in der Subtraktions-Höhenmap zu erhalten (=2. Klassifizierungswert, AvgHeigth1).

Um die Weiterverarbeitung zu beschleunigen, werden nun alle Peaks, deren relative Höhe unter einem definierten Grenzwert (AvgHeight1 *EZThresh) liegt, ausgefilte rt. Danach können der dritte und vierte Wert berechnet werden, die AvgArea2 (Durchschnittliche Fläche der verbliebenen Peaks) und SumArea1 (Grundfläche der verbliebenen Peaks). Im letzten Schritt wird der maximale Höhenwert (also die höchste Spitze im segmentierten Bereich) zur Ermittlung des dritten Klassifizierungswertes herangezogen. Dabei werden alle verbliebenen Spitzen, deren relative Höhe um einen gewissen Prozentsatz niedriger ist als die Maximalhöhe, ebenfalls ausgefiltert. Die verbliebenen höchsten Spitzen werden dann inkl. Ihrer Fläche aufsummiert. Dadurch ergeben sich weitere Klassifizierungswerte, wie die PixelAboveThreshold (Grundfläche der nun verbliebenen Peaks), AvgHeight3 (Durchschnittshöhe der nun verbliebenen Pixel), (siehe Fig. 22).

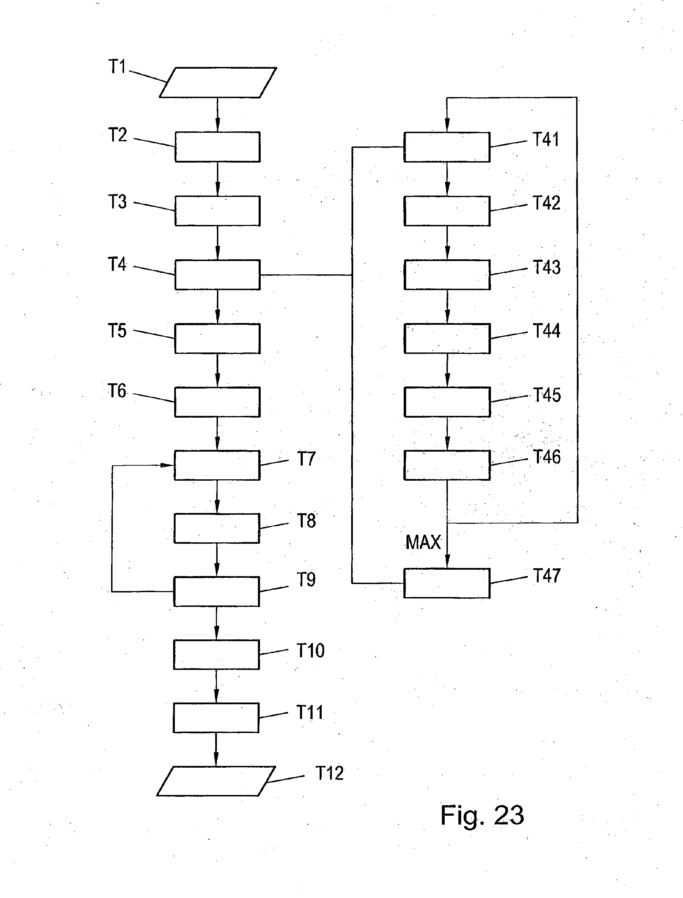

Zur besseren Verständlichkeit sind die ausgeführten Algorithmen noch im Ablaufdiagramm gemäß Fig. 23 dargestellt.

Dazu sei erklärend auf folgende Definitionen der Ergebnis bzw. Parameter bzw. Werte verwiesen:
- AvgMagenta = Durchschnittlicher Magenta-Wert der Pixel innerhalb der Kontur oder des Rechtecks, das die Kontur umschließt
- AvgSaturation = Durchschnittlicher Sättigungs-Wert der Pixel innerhalb der Kontur oder des Rechtecks, das die Kontur umschließt
- kompaktRadius = Verhältnis des Quotienten aus Fläche und Umfang der Kontur im Verhältnis zum durchschnittlichen Radius der Kontur • AvgVolume = Durchschnittlicher Höhenwert aller Punkte die einen größeren Wert als die Mittelwertfläche aufweisen (= Volumen von Additionsmap)
- AvgHeight1 = Durchschnittliche Höhe der Peaks aller gefundenen Peaks
- AvgArea2 = Durchschnittliche Fläche der Peaks, deren Spitzen (RelHeight) über AvgHeight1*EZThresh liegen
- SumArea1 = Grundfläche (PeakArea) jener Peaks, deren Spitzen (RelHeight) über AvgHeight1*EZThresh liegen
- AvgHeight3 = Durchschnittshöhe der Pixel, deren Höhenwert größer als PixeAboveThreshThreshold*(Höhe des höchsten Peaks) ist
- PixelAboveThresh = Anzahl der Pixel über einem bestimmten Schwellwert (abghängig von der höchsten auftretenden Höhe im Segment)

Am Ende werden dann zur Gesamtbestimmung bzw. zur Ermittlung vergleichbarer Klassifizierungen die ermittelten Werte abhängig vom verwendeten Kameratyp kombiniert, dabei sind verschiedene Kombinationen und Verknüpfungsvarianten möglich wie zB das Produkt aus AvgMagenta, AvgSaturation, KompaktRadius, AvgVolume, AvgHeight1 und PixelAboveThreshold oder das Produkt aus AvgMagenta, AvgSaturation, AvgHeight3, AvgVolume, AvgArea2 und SumArea1. Auch andere Kombinationen sind denkbar. Anstatt einer Multiplikation kann auch zumindest teilweise eine Addition der Werte erfolgen.

### Legende Fig. 14

S1: Bildaufnahme
S2: K3ResultData, 400 x 400
S3: Konvertieren CMYK, HSV
S4: Höhensegmentierung
S5: Kalk. InflamationVolume
S6: Kalk. relVolumen
S7: COGinCont = true
S8: Kalk. avgMag & avgSat (Kontur)
S9: Kalk. sur_avgMag & sur_avgSat (Restbild)
S10: Kalk. relMag & relSat
S11: Kal. SK Wert
S12: Auswertung Ende (SK-Wert, rel. Mag, rel. Volume)
S13: Kalk. avgMag & avgSat (Rechteck)
S14: Kalk. sur_avgMag & sur_avgSat (Restbild)
S41: Kalk. Polynommatrix
S42: Kalk. Polynomebene
543: Threshold Polynomebene
S44: Medianfilter, Haarfilter (MF0)
S45: Erstellung Höhengraubild
S46: Segmentierung Magentabild
S47: Erstellung surfaceimg (Magbild + Höhengraubild)
S48: Medianfilter (9)
S49: Erstellung Binärbild durch Tresh mit avgHeight
S50: Erode (2), Dilate (1)
S51: FindContours
S52: MaxAreaContour, boundRect, cog, maxarea
S53: Kalk, COG Surfaceimg
S54: COG_S in boundRect
S55: COGinCont = true
S56: Bounding area (rot/gelb)
S57: COGinCont = false

### Legende zu Fig. 23

T1: Bildaufnahme
T2: K3ResultData, 400x400
T3: Konvertieren CMYK, HSV
T4: Farbsegmentierung
T5: ROI Segment Höhenmap glätten
T6: Erstellung versch. Höhenmaps (AvgVolume)
T7: Maximum (Peak, Gipfel) in Subtrahierter Map suchen
T8: Grenzen und Größe (Fläche) des Peaks bestimmen (AvgHeightl)
T9: Peak-Eigenschaften in Liste schreiben und ganzen Peak "löschen"
T10: Relevante Peaks herausfiltern = EZThresh (AvgArea2, SumArea 1)
T11: Analyse Peaks/Pixel über Schwellwert (PixAbovThreshold) (PixelAboveThresh, AvgHeight 3)
T12: Auswertung Ende
T41: Vorfilter Magentabild
T42: Medianfilter (9)
T43: Erode (2), Dilate (1)
T44: Erstellung Binärbild
T45: FindContours
T46: kompaktRadius, AvgMag, Avg Sat, ROI
T47: kompaktRadius, AvgMag, Avg Sat, ROI

## Patentansprüche

1. Einrichtung zur Ermittlung eines Hautentzündungswertes (Z), mit
- einem opto-elektronischen Messgerät (1), vorzugsweise einem 3D-Scanner, zur Aufnahme eines dreidimensionalen Bildes (B) eines Entzündungsbereiches (E) auf menschlicher oder tierischer Haut (H), wobei durch das opto-elektronische Messgerät (1) flächenbezogene (A), räumliche (V) und farbliche (F) Werte des dreidimensionalen Bildes (B) erfassbar sind,
- einer Recheneinheit (2) zur Berechnung des Hautentzündungswertes (Z) aus den vom Messgerät (1) erfassten flächenbezogenen (A), räumlichen (V) und farblichen (F) Werten und
- einer Anzeigeeinheit (3) zur Anzeige des berechneten Hautentzündungswertes (Z),
**dadurch gekennzeichnet, dass** die Recheneinheit (2) eingerichtet ist, durch Abgrenzung der farblichen Werte (F) der einzelnen Bildpunkte (P) und durch Abgrenzung der räumlichen Werte (V) der einzelnen Bildpunkte (P) die flächenbezogenen Werte (A) des aufgenommenen dreidimensionalen Bildes (B) in einen Entzündungsherd (C) und einen an den Entzündungsherd (C) angrenzenden und diesen umgebenden Herdumgebungsbereich (U) zu unterscheiden, und dass die Einrichtung eingerichtet ist, jedem flächenbezogenem Wert (A) des durch das opto-elektronische Messgerät (1) aufgenommenen dreidimensionalen Bildes (B) einen Magentawert im CMYK-Farbmodell zuzuweisen.

2. Einrichtung nach Anspruch 1, **dadurch gekennzeichnet, dass** das aufgenommene dreidimensionale Bild (B) des Entzündungsbereiches (E) aus einer Vielzahl von rasterförmig in einem dreidimensionalen Koordinatensystem (4) angeordneten Bildpunkten (P) besteht, wobei jeder flächenbezogene Wert (A) einem einzigen, im Koordinatensystem (4) eindeutigen Bildpunkt (P) entspricht.

3. Einrichtung nach Anspruch 1 oder 2, **dadurch gekennzeichnet, dass** jedem flächenbezogenen Wert (A) eines durch das opto-elektronische Messgerät (1) aufgenommenen dreidimensionalen Bildes (B) sowohl ein, vorzugsweise einziger, farblicher Wert (F) als auch ein, vorzugsweise einziger, räumlicher Wert (V) zuweisbar ist.

4. Einrichtung nach Anspruch 1, 2 oder 3, **dadurch gekennzeichnet, dass** jeder räumliche Wert (V) einem Höhenwert des jeweiligen Bildpunkts (P) im dreidimensionalen Koordinatensystem (4) entspricht.

5. Einrichtung nach einem der Ansprüche 1 bis 4, **dadurch gekennzeichnet, dass** durch Vergleich der gemittelten farblichen Werte (F) im Entzündungsherd (C) und der gemittelten farblichen Werte (F) im Herdumgebungsbereich (U) ein relativer Gesamt-Farbwert (FW) des Entzündungsherds (C) ermittelbar ist.

6. Einrichtung nach einem der Ansprüche 1 bis 5, **dadurch gekennzeichnet, dass** aus den räumlichen Werten (V) im Entzündungsherd (C) ein absoluter Gesamt-Volumenwert (VW_{V}) des Entzündungsherds (C) ermittelbar ist.

7. Einrichtung nach einem der Ansprüche 1 bis 6, **dadurch gekennzeichnet, dass** durch Vergleich von gemittelten räumlichen Werten (V) im Entzündungsherd (C) zu gemittelten räumlichen Werten (V) im Herdumgebungsbereich (U) ein relativer Gesamt-Volumenwert (VW_{R}) des Entzündungsherds (C) ermittelbar ist.

8. Einrichtung nach Anspruch 7, **dadurch gekennzeichnet, dass** der relative Gesamt-Volumenwert (VW_{R}) ein Vergleichswert der Oberflächenrauheit im Entzündungsherd (C) zur Oberflächenrauheit im Herdumgebungsbereich (U) ist.

9. Einrichtung nach einem der Ansprüche 1 bis 8, **dadurch gekennzeichnet, dass** ein flächenbezogener Wert (A) einem den Umfang des Entzündungsherdes entsprechenden Umfangswert entspricht und/oder ein flächenbezogener Wert (A) einem die Fläche des Entzündungsherdes (C) repräsentierenden Flächenwert entspricht.

10. Einrichtung nach Anspruch 9, **dadurch gekennzeichnet, dass** ein flächenbezogener Wert (A) in Abhängigkeit vom Flächenwert und vom Umfangswert gebildet ist und einem das Verhältnis von Umfangswert zu Flächenwert repräsentierenden Kompaktheitswert entspricht.

11. Einrichtung nach einem der Ansprüche 1 bis 10, **dadurch gekennzeichnet, dass** ein relativer Gesamt-Volumenwert (VW_{R}) einem die durchschnittliche Höhe aller Erhebungen (G) im Entzündungsherd (C) repräsentierenden durchschnittlichen Höhenwert und/oder einem die Fläche der höchsten Erhebungen (G) repräsentierenden Maximalhöhen-Flächenwert entspricht, wobei die höchsten Erhebungen (G) jene Erhebungen (G) sind, deren Höhe zumindest 70 %, vorzugsweise zumindest 85 %, der Höhe der allerhöchsten Erhebung (GH) beträgt.

## Claims

1. A device for determining a skin inflammation score or value (Z), comprising
- an optoelectronic measuring instrument or device (1), preferably a 3D scanner, for taking a three-dimensional image (B) of an inflammation area (E) on human or animal skin (H), wherein area-related (A), space (V) and color (F) values of the three-dimensional image (B) are detectable by the optoelectronic measuring instrument (1),
- a processing or computing unit (2) for calculating the skin inflammation score (Z) from the area-related (A), space (V) and color (F) values detected by the measuring instrument (1), and
- a display unit (3) for displaying the calculated skin inflammation score (Z),
**characterized in that** the processing unit is arranged to distinguish the area-related values (A) of the scanned three-dimensional image into an inflammation focus (C) and a focus-surrounding area (U) adjoining and surrounding the inflammation focus (C) by delimiting the color values (F) of the individual pixels (P) and by delimiting the space values (V) of the individual pixels (P) and **in that** the device is arranged to assign each area-related value (A) of the three-dimensional image (B) scanned by the optoelectronic measuring instrument (1) a magenta value in the CMYK color model.

2. A device according to claim 1, **characterized in that** the scanned three-dimensional image (B) of the inflammation area (E) is comprised of a multitude of pixels (P) arranged in a three-dimensional coordinate system (4) in grid-like fashion, wherein each area-related value (A) corresponds to a single pixel (P) that is unique in the coordinate system (4).

3. A device according to claim 1 or 2, **characterized in that** both a, preferably single, color value (F) and a, preferably single, space value (V) are assignable to each area-related value (A) of a three-dimensional image (B) scanned by the optoelectronic measuring instrument (1).

4. A device according to claim 1, 2 or 3, **characterized in that** each space value (V) corresponds to a height value of the respective pixel (P) in the three-dimensional coordinate system (4).

5. A device according to any one of the claims 1 to 4, **characterized in that** a relative overall color value (FW) of the inflammation focus (C) is determinable by comparing the averaged color values (F) in the inflammation focus (U) and the averaged color values (F) in the focus-surrounding area (U).

6. A device according to any of the claims 1 to 5, **characterized in that** an absolute overall volume value (VWv) of the inflammation focus (C) is determinable from the space values (V) in the inflammation focus (C).

7. A device according to any one of claims 1 to 6, **characterized in that** a relative overall volume value (VW_{R}) of the inflammation focus (C) is determinable by comparing averaged space values (V) in the inflammation focus (C) to averaged space values (V) in the focus-surrounding area (U).

8. A device according to claim 7, **characterized in that** the relative overall volume value (VW_{R}) is a comparative value of the surface roughness in the inflammation focus (C) to the surface roughness in the focus-surrounding area (U).

9. A device according to any one of claims 1 to 8, **characterized in that** an area-related value (A) corresponds to a peripheral value corresponding to the periphery of the inflammation focus, and/or an area-related value (A) corresponds to an area value representing the surface area of the inflammation focus (C).

10. A device according to claim 9, **characterized in that** an area-related value (A) is formed as a function of the area value and of the peripheral value and corresponds to a compactness value representing the ratio of the peripheral value to the area value.

11. A device according to any one of claims 1 to 10, **characterized in that** a relative overall volume value (VW_{R}) corresponds to an average height value representing the average height of all elevations (G) in the inflammation focus (C) and/or a maximum-height area value representing the surface area of the highest elevations (G), the highest elevations (G) being those elevations whose heights are at least 70%, preferably at least 85%, of the height of the highermost elevation (GH).

## Revendications

1. Système permettant de déterminer une valeur d'inflammation de la peau (Z), avec
- un appareil de mesure optoélectronique (1), de préférence un scanneur 3D, permettant de prendre une image tridimensionnelle (B) d'une zone d'inflammation (E) sur une peau (H) humaine ou animale, des valeurs surfaciques (A), volumiques (V) et colorées (F) de l'image tridimensionnelle (B) pouvant être détectées par l'appareil de mesure optoélectronique (1),
- une unité de calcul (2) permettant de calculer la valeur d'inflammation de la peau (Z) à partir des valeurs surfaciques (A), volumiques (V) et colorées (F) détectées par l'appareil de mesure optoélectronique (1), et
- une unité d'affichage (3) permettant d'afficher la valeur d'inflammation de la peau (Z) calculée,
**caractérisé en ce que** l'unité de calcul (2) est agencée pour différencier, par délimitation des valeurs colorées (F) des différents points d'image (P) et par délimitation des valeurs volumiques (V) des différents points d'image (P), les valeurs surfaciques (A) de l'image tridimensionnelle (B) prise dans un foyer d'inflammation (C) et dans une zone périphérique du foyer (U) limitrophe au foyer d'inflammation (C) et entourant ce dernier, et **en ce que** le système est agencé pour attribuer à chaque valeur surfacique (A) de l'image tridimensionnelle (B) prise par l'appareil de mesure optoélectronique (1) une valeur magenta dans le modèle de couleurs CMYK.

2. Système selon la revendication 1, **caractérisé en ce que** l'image tridimensionnelle (B) prise de la zone d'inflammation (E) se compose d'une pluralité de points d'image (P) disposés sur une grille dans un système de coordonnées tridimensionnelles (4), chaque valeur surfacique (A) correspondant à un seul point d'image (P) univoque dans le système de coordonnées (4).

3. Système selon la revendication 1 ou 2, **caractérisé en ce qu'**aussi bien une valeur colorée (F), de préférence unique, qu'une valeur volumique (V), de préférence unique, peuvent être attribuées à chaque valeur surfacique (A) d'une image tridimensionnelle (B) prise par l'appareil de mesure optoélectronique (1).

4. Système selon la revendication 1, 2 ou 3, **caractérisé en ce que** chaque valeur volumique (V) correspond à une valeur de hauteur du point d'image respectif (P) dans le système de coordonnées tridimensionnelles (4).

5. Système selon l'une quelconque des revendications 1 à 4, **caractérisé en ce que**, par comparaison des valeurs colorées (F) moyennées dans le foyer d'inflammation (C) et des valeurs colorées (F) moyennées dans la zone périphérique du foyer (U), on peut déterminer une valeur de couleur totale (FW) du foyer d'inflammation (C).

6. Système selon l'une quelconque des revendications 1 à 5, **caractérisé en ce que**, à partir des valeurs volumiques (V) dans le foyer d'inflammation (C), on peut déterminer une valeur de volume totale absolue (VW_{V}) du foyer d'inflammation (C).

7. Système selon l'une quelconque des revendications 1 à 6, **caractérisé en ce que**, par comparaison des valeurs volumiques (V) moyennées dans le foyer d'inflammation (C) aux valeurs volumiques (V) moyennées dans la zone périphérique du foyer (U), on peut déterminer une valeur de volume totale relative (VW_{R}) du foyer d'inflammation (C).

8. Système selon la revendication 7, **caractérisé en ce que** la valeur de volume totale (VW_{R}) est une valeur de comparaison de la rugosité à la surface dans le foyer d'inflammation (C) par rapport à la rugosité à la surface dans la zone périphérique du foyer (U).

9. Système selon l'une quelconque des revendications 1 à 8, **caractérisé en ce qu'**une valeur surfacique (A) correspond à une valeur de circonférence correspondant à la circonférence du foyer d'inflammation et/ou une valeur surfacique (A) correspond à une valeur de surface représentant la surface du foyer d'inflammation (C).

10. Système selon la revendication 9, **caractérisé en ce qu'**une valeur surfacique (A) est formée en fonction de la valeur de surface et de la valeur de circonférence et correspond à une valeur de compacité représentant le rapport de la valeur de circonférence sur la valeur de surface.

11. Système selon l'une quelconque des revendications 1 à 10, **caractérisé en ce qu'**une valeur de volume totale relative (VW_{R}) correspond à une valeur de hauteur moyenne représentant la hauteur moyenne de toutes les élévations (G) dans le foyer d'inflammation (C) et/ou à une valeur de surface de hauteur maximale représentant la surface des élévations (G) les plus hautes, les élévations (G) les plus hautes étant les élévations (G) dont la hauteur est d'au moins 70 %, de préférence 85 %, de la hauteur de l'élévation la plus haute entre toutes (GH).
